# EUROPEAN PATENT APPLICATION

(11) **EP 4 851 876 A1**
(43) Date of publication of application: **09.12.2026**
(21) Application number: 25748723.1
(22) Date of filing: 30.01.2025
(51) Int. Cl.: G06N 20/00, A61B 3/113, G06F 18/213, G06N 5/04, G06N 5/025, G06F 123/02

(54) **MODEL GENERATION METHOD AND INFERENCE PROGRAM**

(30) Priority: 02.02.2024 JP 2024014867
(71) Applicant: National Institute of Information and Communications Technology, Koganei-shi, Tokyo 184-8795 (JP)
(72) Inventor: YOKOI, Atsushi, Koganei-shi, Tokyo 184-8795 (JP); NISHIMOTO, Shinji, Koganei-shi, Tokyo 184-8795 (JP)
(74) Representative: Grünecker Patent- und Rechtsanwälte PartG mbB
(86) International application number: PCT/JP2025/002995
(87) International publication number: WO 2025/164720

(57) **Abstract**

A model generation apparatus according to one aspect of the present disclosure acquires eye-related data measured from a subject viewing content, uses the acquired eye-related data to perform machine learning of an inference model, and outputs a result of the machine learning. The machine learning includes training the inference model to acquire, from the eye-related data, the ability to infer a semantic representation in an information space corresponding to substance included in the viewed content. Accordingly, the present disclosure provides a technique that enables substance, which is perceived by an individual while viewing content, to be inferred in a simple and cost-effective manner.

## Description

### [Technical Field]

The present invention relates to a model generation method and an inference program.

### [Background Art]

The question of how to quantify the impact of diverse content on diverse individuals, and how to optimize content for the diverse individuals, is a critical issue across a wide range of fields including, for instance, education, entertainment, advertising, and public policy.

With the rapid advancement of artificial intelligence technology including large language models, methods are being developed that quantitatively represent substance, which is included in content expressed by a wide range of modalities, as fixed-length vectors in any latent spaces such as image feature spaces, semantic spaces, and conceptual spaces. For example, NPL 1 proposes an architecture for computing continuous vector representations of words from large-scale datasets.

In addition, with recent advancements in techniques for quantitatively modeling brain activity, technology that enables substance of perceptual experiences and the like to be interpreted as vectors in a latent space is also being realized. For example, PTL 1 proposes an estimation method of estimating perceived semantic substance by measuring brain activity and analyzing the measured brain activity.

### [Citation List]

### [Patent Literature]

[PTL 1] Japanese Patent Application Laid-open No. 2016-195716

### [Non Patent Literature]

[NPL 1] Tomas Mikolov, Kai Chen, Greg Corrado, Jeffrey Dean, "Efficient estimation of word representations in vector space", [online], [retrieved January 29, 2024], Internet <URL:https://arxiv.org/abs/1301.3781>

### [Summary of Invention]

### [Technical Problem]

In information analysis methods based on brain activity such as those proposed in PTL 1 and the like, large-scale measurement apparatuses such as fMRI (functional magnetic resonance imaging) are used to measure brain activity. Such large-scale measurement apparatuses are expensive and highly restrictive. Consequently, there is a problem in that it is difficult to infer substance perceived by an individual in a simple and cost-effective manner.

In one aspect, the present invention has been made in light of such circumstances, and an object thereof is to provide a technique that enables substance, which is perceived by an individual while viewing content, to be inferred in a simple and cost-effective manner.

### [Solution to Problem]

To solve the problems described above, the present invention adopts the following configurations. Note that the following configurations can be combined as appropriate.

A model generation method according to one aspect of the present invention is an information processing method including executing by a computer a step of acquiring eye-related data measured from a subject viewing content; a step of using the acquired eye-related data to perform machine learning of an inference model; and a step of outputting the results of the machine learning. The machine learning includes training the inference model so as to acquire the ability to infer, from the eye-related data, a semantic representation in an information space corresponding to substance included in the viewed content.

The present inventors found that, through an experimental example described below, it is possible to infer substance perceived by an individual while viewing content based on eye activity (in other words, eye-related data can be used as an alternative signal source for brain activity data). Based on this finding, in this configuration, a trained inference model (trained model) having acquired the ability to infer, from the eye-related data, substance perceived from the content is generated. Compared to brain activity, eye activity can be measured in a simple and cost-effective manner. Therefore, according to the configuration, by using the generated trained model, substance perceived by an individual while viewing content can be inferred in a simple and cost-effective manner.

In the model generation method according to the aspect described above, the computer may further execute a step of acquiring behavioral data indicating the behavior of the subject viewing the content. Acquired behavioral data may be further used for the machine learning of the inference model. In the machine learning, the inference model may be trained to acquire the ability to infer the semantic representation from the eye-related data and the behavioral data.

For example, actions taken while viewing content such as searching for terms that appear in the content or cheering for athletes featured in the content may be prompted as a result of perceiving the content. In other words, since behavior while viewing content is correlated with perceptual consequences, behavioral data of an individual viewing content can serve as a clue for inferring perceptual content. According to the configuration, a trained model that can further accept input of behavioral data can be generated. According to the generated trained model, by using behavioral data together with eye-related data as explanatory variables, an improvement in the accuracy of inferring substance perceived by an individual while viewing content can be expected.

In the model generation method according to the aspect described above, the acquired eye-related data may be constituted of gaze data, blinking data, pupil diameter data, or a combination thereof. A gaze, blinking, and pupil diameter accurately represent eye activity. Therefore, according to the configuration, by using at least any of gaze data, blinking data, and pupil diameter data as eye-related data, a trained model having acquired the ability to properly infer perceptual substance from eye activity can be generated.

In addition, the form of the present invention need not be limited to the stage of model generation described above. The form of the present invention may relate to an inference stage that uses a trained inference model generated by the model generation method described above. For example, one aspect of the present invention may be an inference program that uses a trained inference model generated by the model generation method described above.

An inference program according to one aspect of the present invention is a program causing a computer to execute: a step of acquiring eye-related data measured from a subject viewing object content; a step of using a trained inference model to infer a semantic representation in information space corresponding to substance, which is included in the object content, from the acquired eye-related data; and a step of outputting the results of inferring the semantic representation. According to the configuration, substance perceived by an individual while viewing content can be inferred in a simple and cost-effective manner.

The inference program according to the one aspect described above may cause the computer to further execute a step of acquiring behavioral data indicating the behavior of the subject viewing the object content. In the step of inferring semantic representation, the computer may be caused to infer the semantic representation from the acquired eye-related data and the acquired behavioral data. According to the configuration, an improvement in the accuracy of inferring substance perceived by an individual (subject) can be expected.

Note that the form of the present invention need not be limited to the model generation method described above. In one aspect of the present invention, as an alternative aspect of the model generation method according to each aspect described above, one aspect of the present invention may be an information processing apparatus (model generation apparatus) that realizes all of or a part of each configuration described above, a program, or a storage medium readable by a machine such as a computer, storing such a program. The storage medium readable by a machine such as a computer refers to a medium that stores information such as programs through electrical, magnetic, optical, mechanical, or chemical action. In addition, the form of the present invention need not be limited to the inference program described above. As an alternative aspect of the inference program according to each aspect described above, one aspect of the present invention may be an information processing apparatus (inference apparatus) that realizes all of or a part of each configuration described above, an information processing method (inference method), a storage medium readable by a machine such as a computer, storing the program.

For example, the model generation apparatus according to one aspect of the present invention may include a control unit. The control unit may be configured to execute the steps of: acquiring eye-related data measured from a subject viewing content; performing machine learning of an inference model using the acquired eye-related data; and outputting the results of the machine learning. The machine learning may include training the inference model to acquire the ability to infer, from the eye-related data, a semantic representation in an information space corresponding to substance included in the viewed content.

For example, the inference apparatus according to one aspect of the present invention may include a control unit. The control unit may be configured to execute the steps of: acquiring eye-related data measured from a subject viewing object content; inferring, using a trained inference model, semantic representation in information space corresponding to substance included in the object content from the acquired eye-related data; and outputting the results of inferring the semantic representation.

For example, the inference method according to one aspect of the present invention may be an information processing method in which a computer executes the steps of: acquiring eye-related data measured from a subject viewing object content; inferring, using a trained inference model, semantic representation in information space corresponding to substance included in the object content from the acquired eye-related data; and outputting the results of inferring the semantic representation.

### [Advantageous Effect of Invention]

According to the present invention, a technique that enables substance perceived by an individual while viewing content to be inferred in a simple and cost-effective manner can be provided.

### [Brief Description of Drawings]

[Fig. 1]

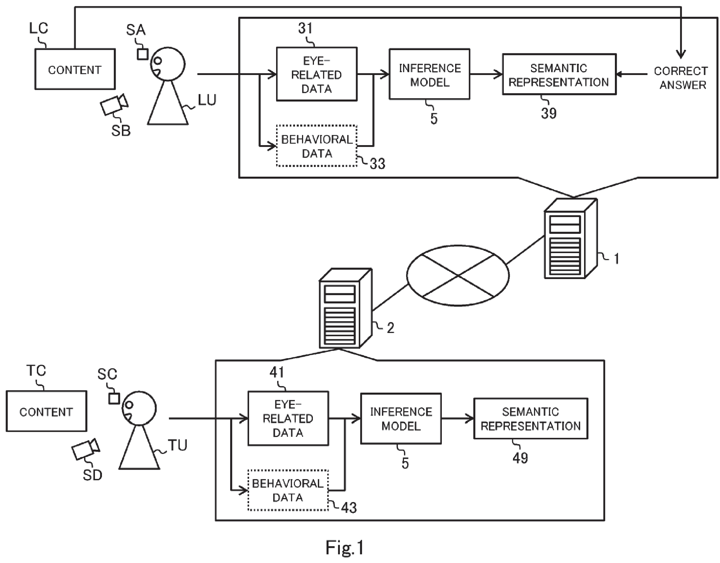

   Fig. 1 schematically shows an example of a situation in which the present invention is applied.
[Fig. 2]

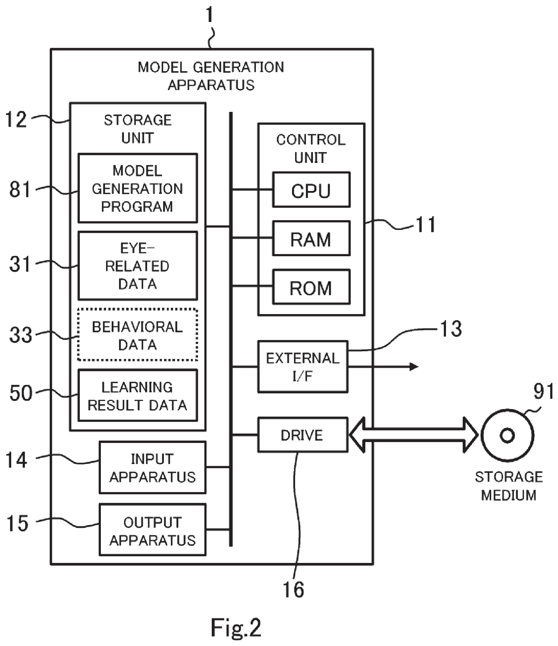

   Fig. 2 schematically shows an example of a hardware configuration of a model generation apparatus.
[Fig. 3]

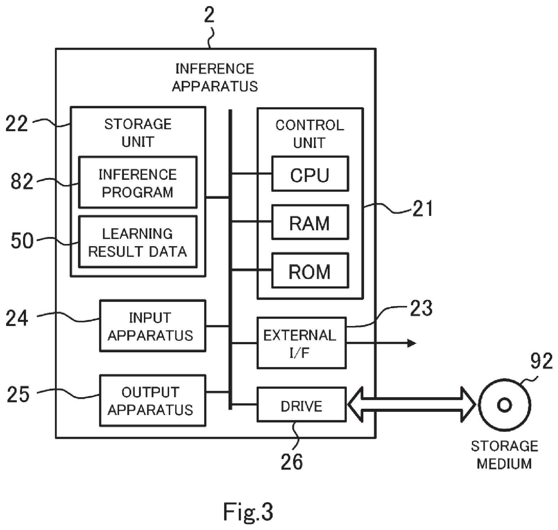

   Fig. 3 schematically shows an example of a hardware configuration of an inference apparatus.
[Fig. 4]

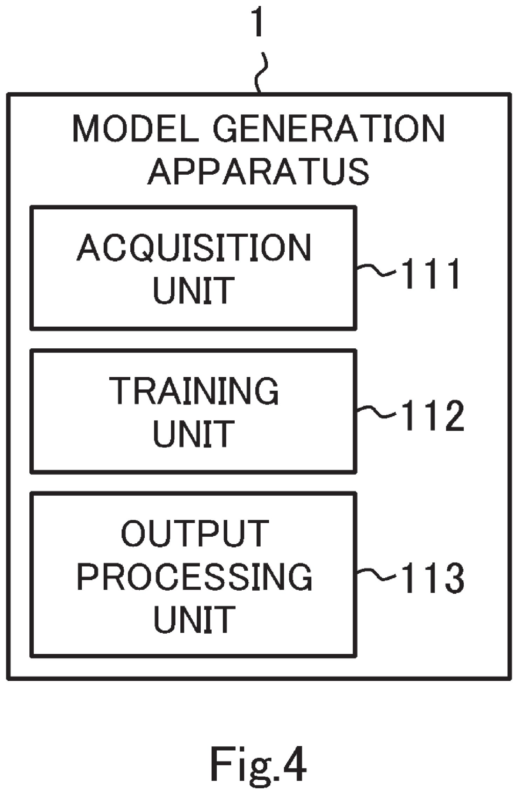

   Fig. 4 schematically shows an example of a software configuration of the model generation apparatus.
[Fig. 5]

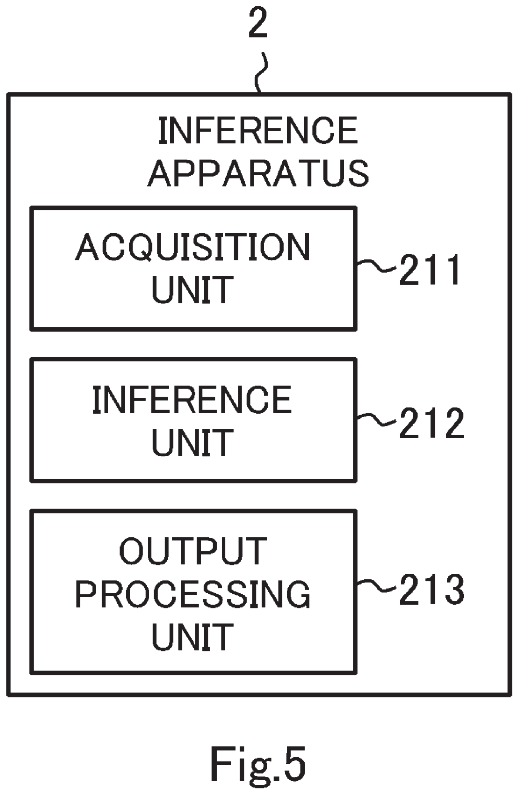

   Fig. 5 schematically shows an example of a software configuration of the inference apparatus.
[Fig. 6]

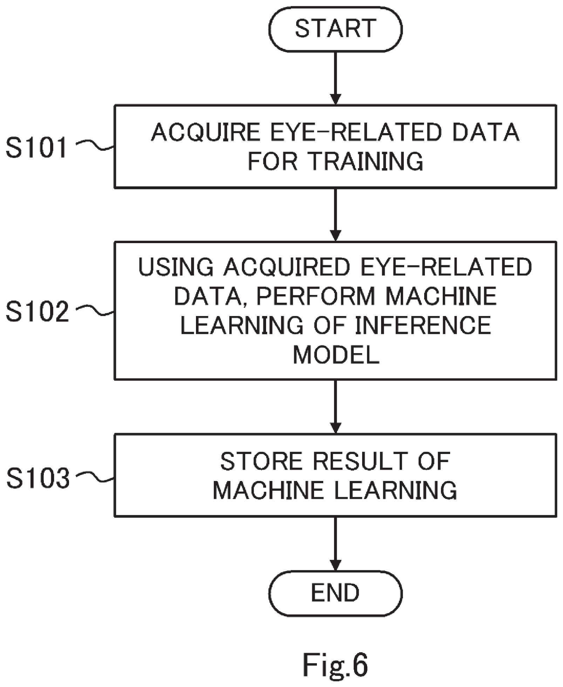

   Fig. 6 is a flow chart showing an example of a processing procedure of the model generation apparatus.
[Fig. 7]

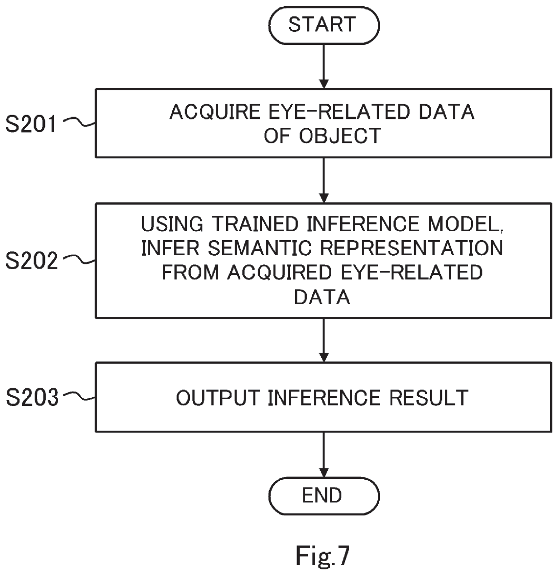

   Fig. 7 is a flow chart showing an example of a processing procedure of the inference apparatus.
[Fig. 8]

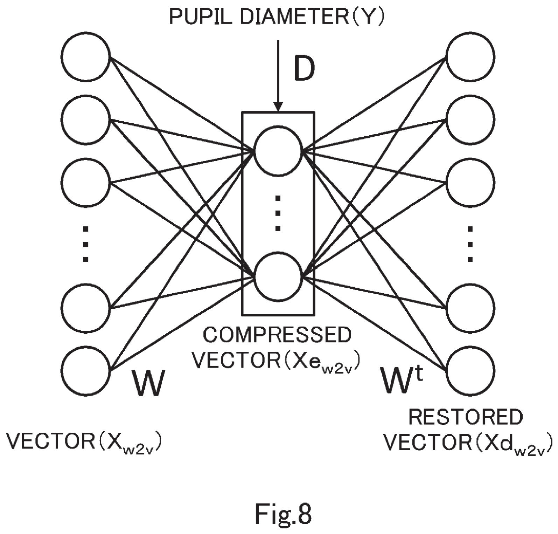

   Fig. 8 shows a relationship between a principal component vector and a decoding model obtained from an experimental example.
[Fig. 9]

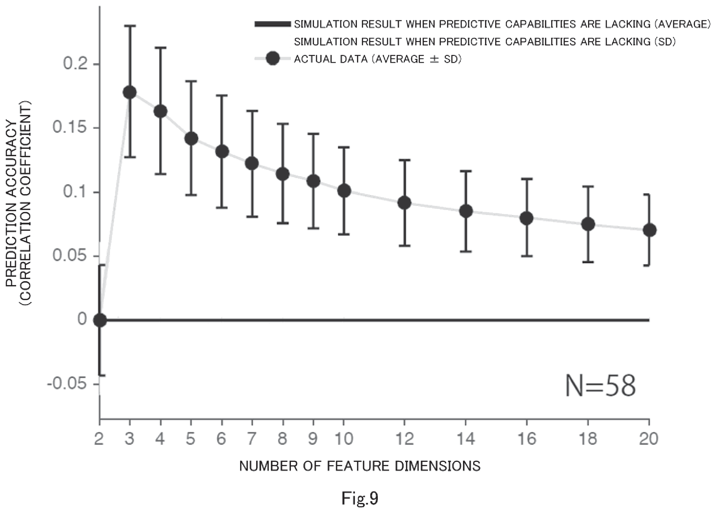

   Fig. 9 shows a result of calculating an average value of inference accuracy of an inference model in the experimental example for each dimension of the principal component vector.
[Fig. 10]

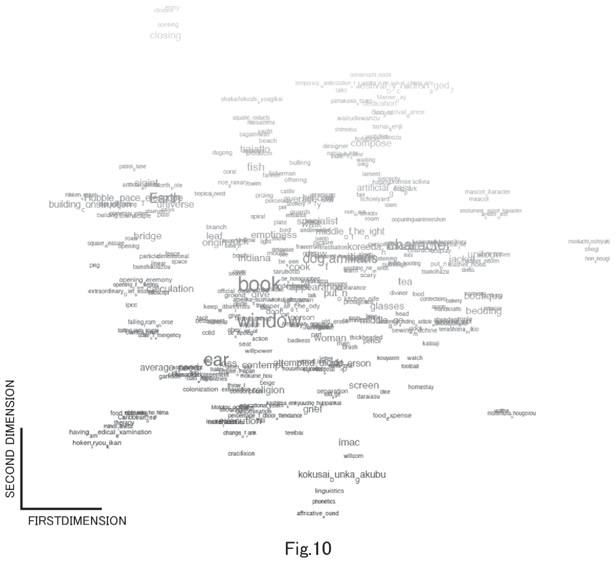

   Fig. 10 shows a result of representing, using t-SNE, perceptual substance inferred from a pupil diameter.
[Fig. 11]

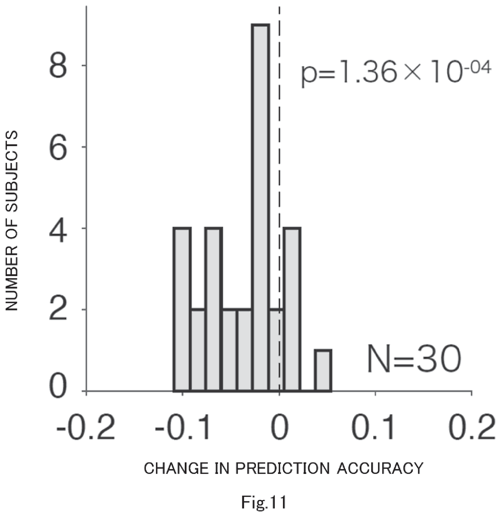

   Fig. 11 shows a result of calculating a change in inference accuracy of an encoding model among individuals in the experimental example.

### [Description of Embodiments]

Hereinafter, an embodiment according to one aspect of the present invention (hereinafter, also described as "present embodiment") will be described based on the drawings. However, the present embodiment described below is merely an example of the present invention in all respects. It is needless to say that various improvements and modifications may be made without departing from the scope of the present invention. In other words, when implementing the present invention, specific configurations corresponding to embodiments may be adopted as appropriate. Note that while data described in the present embodiment is explained in natural language, more specifically, the data is specified in computer-readable pseudo-language, commands, parameters, machine code, and the like.

### §1 Example of Application

Fig. 1 schematically shows an example of a situation in which the present invention is applied. A system according to the present embodiment includes a model generation apparatus 1 and an inference apparatus 2. The model generation apparatus 1 is one or more computers configured to generate a trained inference model 5 by controlling implementation of machine learning. The inference apparatus 2 is one or more computers configured to infer perceptual substance of a subject TU using the trained inference model 5.

The model generation apparatus 1 according to the present embodiment acquires eye-related data 31 for training measured from a subject LU viewing content LC for training. The content LC for training may also be referred to as training content. The model generation apparatus 1 uses the acquired eye-related data 31 to perform machine learning of the inference model 5. The machine learning includes training the inference model 5 to acquire, from the eye-related data 31, the ability to infer a semantic representation 39 in an information space corresponding to substance included in the content LC. The semantic representation in information space corresponds to perceptual content with respect to content. Therefore, according to the machine learning, the trained inference model 5 having acquired the ability to infer perceptual substance from eye-related data measured during viewing of content can be generated. The model generation apparatus 1 outputs the result of machine learning. The generated trained inference model 5 may be provided to the inference apparatus 2 by any method and at any timing.

On the other hand, the inference apparatus 2 according to the present embodiment acquires object eye-related data 41 measured from the subject TU viewing object content TC. The inference apparatus 2 infers, using the trained inference model 5, a semantic representation 49 in an information space corresponding to substance included in the object content TC from the acquired eye-related data 41. The semantic representation 49 corresponds to substance perceived by the subject TU with respect to the object content TC. The inference apparatus 2 outputs the result of inferring the semantic representation 49.

It was found that, through an experimental example described below, it is possible to infer substance perceived by an individual while viewing content based on eye activity. Based on this finding, in the model generation apparatus 1 according to the present embodiment, the trained inference model 5 having acquired the ability to infer, from the eye-related data, substance perceived from the content is generated. By using the trained inference model 5, substance perceived by an individual while viewing content can be inferred in a simple and cost-effective manner. In the inference apparatus 2 according to the present embodiment, substance perceived by the subject TU while viewing the object content TC can be inferred in a simple and cost-effective manner.

### (Content)

In one example, content (content LC, object content TC) may be any information viewable by a human. Viewing may involve at least one of watching and listening. As long as the content is viewable, a data format of the content need not be particularly limited and may be selected as appropriate according to the embodiment. For example, the content (content LC, object content TC) may be constituted of text data, image data (video, still image), sound data, other numerical data, or a combination of these pieces of data. As a typical example, the content may be constituted of video content that includes audio (such as video content for educational or medical support purposes). Note that the content LC for training and the object content TC may be the same or may differ from each other.

### (Eye-Related Data)

In one example, eye-related data (eye-related data 31, eye-related data 41) may be configured to represent eye activity of a person (subject LU, subject TU) while the person is viewing content. The activity may include a state. The eye activity may be represented by, for example, a gaze, blinking, a pupil diameter, or the like. As long as the eye-related data is configured as described above, a configuration of the eye-related data need not be particularly limited and may be selected as appropriate according to the embodiment.

In one example, the eye-related data (eye-related data 31, eye-related data 41) may be constituted of gaze data, blinking data, pupil diameter data, or a combination thereof. A gaze, blinking, and pupil diameter accurately represent eye activity. Therefore, according to one example of the present embodiment, generation of the trained inference model 5 having acquired the ability to properly infer perceptual substance from eye activity can be expected in the model generation apparatus 1. In addition, by using the trained inference model 5, proper inference of perceptual substance of the subject TU with respect to the object content TC can be expected in the inference apparatus 2.

Eye-related data may be generated by measuring eye activity of a person using any sensor. When collecting training data, a sensor SA may be used to collect training samples of the eye-related data 31 as appropriate. When making an inference, a sensor SC may be used to acquire samples of the eye-related data 41. As long as eye activity can be measured, a type of the sensor (sensor SA, sensor SC) need not be particularly limited and may be selected as appropriate according to the embodiment. In one example, the sensor (sensor SA, sensor SC) may include an imaging apparatus such as an infrared camera. The sensor (sensor SA, sensor SC) may further include an apparatus to assist measurement such as an infrared projector.

A data format of the eye-related data need not be particularly limited and may be selected as appropriate according to the type of sensor or the like and to the embodiment. In addition, at least a part of the eye-related data may be constituted of sensing data obtained by a sensor or analysis data (analysis results) obtained by analyzing the sensing data. For example, the analysis may include extraction of features and the calculation of feature amounts. The extraction of features may include determining a direction of gaze, measuring blinks, and measuring a pupil diameter. Feature amounts may include statistical measures such as a maximum, a minimum, a median, a mean, a variance, a standard deviation, and an n-th percentile.

The model generation apparatus 1 may directly acquire data from the sensor SA or may indirectly acquire the data via another computer. When directly acquiring the data from the sensor SA, the sensor SA may be connected to the model generation apparatus 1. When indirectly acquiring the data via another computer, the model generation apparatus 1 may acquire object data from the other computer via a network, a storage medium, or the like. The model generation apparatus 1 may acquire data measured in the past by the sensor SA or acquire data measured in real-time by the sensor SA.

In a similar manner, the inference apparatus 2 may directly acquire data from the sensor SC or may indirectly acquire the data via another computer. When directly acquiring the data from the sensor SC, the sensor SC may be connected to the inference apparatus 2. When indirectly acquiring the data via another computer, the inference apparatus 2 may acquire object data from the other computer via a network, a storage medium, or the like. The inference apparatus 2 may acquire data measured in the past by the sensor SC or acquire data measured in real-time by the sensor SC.

### (Other Data)

In one example, data other than eye-related data may be further used to infer semantic representation. Fig. 1 illustrates a situation where behavioral data indicating actions taken while viewing content is further used to infer semantic representation, as an example of other data.

In other words, in the example in Fig. 1, the model generation apparatus 1 may be configured to further execute acquisition of behavioral data 33 indicating behavior of the subject LU viewing the content LC. The acquired behavioral data 33 may be further used for the machine learning of the inference model 5. Accordingly, in the machine learning, the inference model 5 may be trained to acquire the ability to infer the semantic representation 39 from the eye-related data 31 and the behavioral data 33.

As described above, for example, actions taken while viewing content such as searching for terms that appear in the content or cheering for athletes featured in the content may be prompted as a result of perceiving the content. In other words, since behavior while viewing content is correlated with perceptual consequences, behavioral data of an individual viewing content can serve as a clue for inferring perceptual substance. Therefore, according to one example of the present embodiment, by generating the trained inference model 5 that further accepts input of behavioral data, an improvement in accuracy of inference of semantic representation in the generated trained inference model 5 can be expected.

In addition, in one example, the inference apparatus 2 may be configured to further execute acquisition of behavioral data 43 indicating behavior of the subject TU viewing the object content TC. In the step of inferring semantic representation, the inference apparatus 2 may infer the semantic representation 49 from the acquired eye-related data 41 and the acquired behavioral data 43. According to one example of the present embodiment, by further incorporating the behavioral data 43 into the inference of the semantic representation 49, an improvement in accuracy of inference can be expected.

Note that in one example, behavioral data (behavioral data 33, behavioral data 43) may be configured to include information regarding behaviors that may be related to the perception of content. As long as the behavioral data is configured as described above, a configuration of the behavioral data need not be particularly limited and may be determined as appropriate according to the embodiment. Behavioral data may be generated by measuring the behavior of a person using any sensor. When collecting behavioral data, a sensor SB may be used to collect training samples of the behavioral data 33 as appropriate. When making an inference, a sensor SD may be used to acquire samples of the behavioral data 43.

As long as the behavior of a person can be measured, a type of the sensor (SB, SD) need not be particularly limited and may be selected as appropriate according to the embodiment. In one example, object behavior to be observed during viewing of content may include, for example, terminal operations (keyboard operations, mouse operations, and the like), physical movements (gestures and the like), vocalizations (including utterances), and vital signs (body temperature, heart rate, pulse, blood pressure, electrocardiogram, electromyogram, perspiration, activity levels, and the like). Accordingly, the sensor (SB, SD) may include a computer, an imaging apparatus, an accelerometer, an angular accelerometer, a gyro sensor, a motion capture system, a microphone, and vital sign sensors. The number of modalities in behavioral data may be selected as appropriate according to the embodiment.

Information regarding terminal operation may be acquired as appropriate from any computer (user terminal). When collecting training data, if the model generation apparatus 1 is to be used as a user terminal and the behavioral data 33 is configured so as to include information regarding terminal operation, the model generation apparatus 1 may be used as a computer (sensor SB) for acquiring the information regarding terminal operation. In a similar manner, the inference apparatus 2 may be used as a computer (sensor SD) for acquiring the information regarding terminal operation. In addition, the vital sign sensors may include thermometers, heart rate monitors, pulse oximeters, blood pressure monitors, electrocardiographs, electromyographs, skin conductance meters, and activity trackers. The vital sign sensors may be configured as wearable terminals such as smartwatches.

A data format of the behavioral data need not be particularly limited and may be selected as appropriate according to the type of sensor or the like and to the embodiment. In addition, at least a part of the behavioral data may be constituted of sensing data obtained by a sensor or analysis data (analysis results) obtained by analyzing the sensing data. In a similar manner to the eye-related data described above, for example, the analysis may include extraction of features and the calculation of feature amounts. The extraction of features may include determining substance of an operation, estimating gestures, and estimating vital signs.

The model generation apparatus 1 may directly acquire data from the sensor SB or may indirectly acquire the data via another computer. When directly acquiring the data from the sensor SB, the sensor SB may be connected to the model generation apparatus 1. When acquiring the data via another computer, the model generation apparatus 1 may acquire object data from the other computer via a network, a storage medium, or the like. The model generation apparatus 1 may acquire data measured in the past by the sensor SB or acquire data measured in real-time by the sensor SB. As described above, the model generation apparatus 1 may also serve as at least a portion of the sensor SB.

In a similar manner, the inference apparatus 2 may directly acquire data from the sensor SD or may indirectly acquire the data via another computer. When directly acquiring the data from the sensor SD, the sensor SD may be connected to the inference apparatus 2. When acquiring the data via another computer, the inference apparatus 2 may acquire object data from the other computer via a network, a storage medium, or the like. The inference apparatus 2 may acquire data measured in the past by the sensor SD or acquire data measured in real-time by the sensor SD. The inference apparatus 2 may also serve as at least a portion of the sensor SD.

The sensor that measures eye-related data (sensor SA, sensor SC) and the sensor that measures behavioral data (sensor SB, sensor SD) may differ from each other or may overlap at least partially. When the sensor that measures eye-related data and the sensor that measures behavioral data overlap at least partially, the eye-related data may include at least a part of behavioral data. For example, when measuring eye activity and behavior of a person using an imaging apparatus, image data obtained by the imaging apparatus may serve at least partially as eye-related data and behavioral data.

Note that a form of inferring semantic representation need not be limited to such examples. In another example, data other than behavioral data may be used to infer semantic representation in addition to or in place of behavioral data. Alternatively, in another example, inference of semantic representation may be performed using only eye-related data.

### (Inference)

In one example, inference may include at least one of identification and regression. In addition, substance of content up to a certain time point may have a correlation with substance of content from that time point onward. Therefore, inference may include predicting the future.

### (Information Space/Semantic Representation)

In one example, information space may be configured such that substance included in content is represented as vectors. A vector is made up or one or more numerical values. A data format of a vector may be selected arbitrarily. In one example, a vector may simply be numerical data. Substance represented by information space may correspond to the meaning of a phrase, a context, or the like.

A phrase may be, for example, a word or the like and may correspond to the meaning at one time point. A phrase may be directly included in content or may be indirectly derived from content. Phrases indirectly derived from content may include, for example, a phrase associated with substance of the content, a phrase derived from interpreting substance of the content, and the like. A phrase may be represented as a vector at one point in information space.

A context may correspond to a sequence of meanings across two or more time points, such as in a text or a scene from a video. In a similar manner to a phrase, a context may also be directly included in content or may be indirectly derived from content. A context may be represented as a vector at one or more points in information space. In one example, the information space may be divided into clusters. Each cluster may be configured to represent a context. Each point in each cluster may be configured to represent a phrase. Accordingly, semantic representation can be distinguished for each context.

In one example, semantic representation (semantic representation 39, semantic representation 49) may be a vector in information space and may represent substance included in content. A relationship between semantic representation and substance of content may be determined as appropriate according to the embodiment. In one example, semantic representation may directly represent content. For example, a numerical representation (binary representation or the like) of a phrase such as a work may be used as semantic representation. In this case, when performing machine learning, a correct answer for semantic representation 39 may be obtained directly from the content LC. In another example, semantic representation may indirectly represent substance of content. For example, semantic representation may be obtained using any projection method for transforming content into a vector representation such as the method proposed in NPL 1. In this case, the information space may also be referred to as a latent space. When performing machine learning, a correct answer for semantic representation 39 may be obtained by projecting the content LC using a predetermined projection method.

Note that the projection method need not be particularly limited and may be selected as appropriate according to the embodiment. For example, projection of content to semantic representation may be performed using a trained machine learning model. The machine learning model may have any architecture such as Transformer or Vision Transformer. Known methods may be adopted as the projection method. In addition, in one example, content may be transformed into a vector representation by a single projection. In another example, content may be transformed into a vector representation by a plurality of projections, for example, by first performing an operation (image2txt) that transforms images into text, followed by executing an operation (word2vec) that transforms words (text) into vectors in a distributed manner.

When making an inference, semantic representation 49 corresponds to substance perceived by the subject TU with respect to the object content TC. As described above, the perceived substance may include, for example, substance directly perceived from content and substance indirectly derived from content, and the like. Substance indirectly derived from content may include, for example, substance associated with the content, substance derived from interpreting substance of the content, and the like. The perceived substance may include substance that is predicted to appear at a future time point based on substance up to the present time point. In other words, perception may include prediction.

### (Inference Model)

The inference model 5 is configured to infer semantic representation from given input (such as eye-related data). As long as such inference processing can be executed, the configuration of the inference model 5 need not be particularly limited and may be determined as appropriate according to the embodiment. The inference model 5 may be constituted of any machine learning model.

The machine learning model is configured to have one or more computation parameters that can be adjusted through machine learning. The one or more computation parameters are used in computation for the intended inference (in the present embodiment, the inference of semantic representation). The machine learning model may be constituted of, for example, neural networks, regression models, decision tree models, support vector machines, and other mathematical formulas (computational models). The method of machine learning may be selected as appropriate according to the machine learning model to be adopted.

In one example, the inference model 5 may include a neural network. Note that a structure of the neural network need not be particularly limited and may be determined as appropriate according to the embodiment. The structure of the neural network may be defined, for example, by the number of layers from an input layer to an output layer, the type of each layer, the number of nodes (neurons) in each layer, and the connections between nodes in each layer. In one example, the neural network may include any mechanism such as recursive structures, self-attention mechanisms, and autoregressive models. In addition, the neural network may include any layers, such as fully connected layers, convolutional layers, pooling layers, deconvolutional layers, unpooling layers, normalization layers, dropout layers, and LSTM (Long short-term Memory) layers. The neural network may include any type of model such as diffusion models, transformer models, and generative models. The neural network may include models capable of learning within context, such as large language models (LLMs) and large vision-language models (LVLMs). For example, by incorporating a self-attention mechanism and an autoregressive model, the neural network can acquire the ability to learn within context. Connection weights between nodes and a threshold of each node are examples of computation parameters. A data format of input and output to and from the inference model 5 need not be particularly limited and may be selected as appropriate according to the embodiment.

Machine learning (in other words, training the inference model 5) involves using training samples to adjust (optimize) the values of computation parameters. Typically, the model generation apparatus 1 may execute supervised learning using a plurality of learning datasets, each learning dataset constituted of a combination of input samples (training samples) and output samples (teaching signals, labels), as part of machine learning processing. An input sample is a sample of input data such as the eye-related data 31 and the behavioral data 33. An output sample is a correct answer of semantic representation 39 corresponding to the input sample. As described above, the correct answer of semantic representation 39 may be obtained directly from the input sample, transforming the input sample using a predetermined projection method, or the like. In supervised learning, values of computation parameters of the machine learning model may be adjusted so that output obtained from the machine learning model when the input sample is given matches a corresponding output sample. Note that the method of generating a trained model need not be limited to such an example and may be modified as appropriate according to the embodiment. The learning dataset need not be limited to the example described above and may be selected as appropriate according to the embodiment. For example, when enabling the ability to learn within context to be acquired, data other than the above may be used as the learning dataset. In addition, the learning method need not be limited to supervised learning and, for example, other methods such as unsupervised learning (including self-supervised learning) and reinforcement learning may be used.

In addition, the input/output format of the inference model 5 need not be particularly limited and may be determined as appropriate according to the embodiment. In one example, the input data may be given to the inference model 5 as it is or may be given after being subjected to preprocessing. Furthermore, in one example, the output of the inference model 5 may be configured to directly or indirectly represent semantic representation (semantic representation 39, semantic representation 49). When the output of the inference model 5 is configured to indirectly represent semantic representation, the semantic representation may be obtained by subjecting the output of the inference model 5 to any information processing (interpretation processing or the like).

Note that the processing of machine learning need not necessarily be executed in the model generation apparatus 1. The model generation apparatus 1 performing machine learning on the inference model 5 may include executing the processing of machine learning in the model generation apparatus 1 as well as instructing a computer other than the model generation apparatus 1 to execute the processing of machine learning. In the latter case, in one example, the model generation apparatus 1 may be connected to other computers as appropriate and may cause the other computers to execute processing of machine learning while exchanging data.

Furthermore, an experimental example to be described later suggests that, although there are individual differences, semantic representations can be inferred even using trained models generated from eye-related data of other people. Therefore, the subject LU in the training stage and the subject TU in the inference stage need not necessarily be the same. The subject LU and the subject TU may be the same person or may be different people. In other words, in the inference processing of semantic representation 49 with respect to the subject TU, the inference apparatus 2 may use the trained inference model 5 generated using data (eye-related data 31 and the like) obtained from the subject TU or the trained inference model 5 generated using data obtained from a person other than the subject TU. In addition, the trained inference model 5 may be generated from data (eye-related data 31 and the like) obtained from a plurality of subjects LU or generated from date obtained from a single subject LU. In one example, the subjects LU and the subjects TU may be classified into groups and the trained inference model 5 may be shared within the same group.

### (System Configuration)

In one example, as shown in Fig. 1, the model generation apparatus 1 and the inference apparatus 2 may be connected to each other via a network. The type of network may be selected as appropriate from, for example, the Internet, wireless communication networks, mobile communication networks, telephone networks, private networks, and the like. However, a method of exchanging data between the model generation apparatus 1 and the inference apparatus 2 need not be limited to such an example and may be selected as appropriate according to the embodiment. In another example, data may be exchanged using a storage medium.

In addition, in the example shown in Fig. 1, the model generation apparatus 1 and the inference apparatus 2 are separate computers. However, a configuration of the system according to the present embodiment need not be limited to such an example and may be determined as appropriate according to the embodiment. In another example, the model generation apparatus 1 and the inference apparatus 2 may be constituted of an integrated computer. In yet another example, at least any of the model generation apparatus 1 and the inference apparatus 2 may be constituted of a plurality of computers.

### §2 Configuration Example

### [Hardware Configuration]

### (Model Generation Apparatus)

Fig. 2 schematically shows an example of a hardware configuration of the model generation apparatus 1 according to the present embodiment. In one example of the present embodiment, the model generation apparatus 1 is a computer in which a control unit 11, a storage unit 12, an external interface 13, an input apparatus 14, an output apparatus 15, and a drive 16 are electrically connected.

The control unit 11 includes a CPU (Central Processing Unit) that is a hardware processor, a RAM (Random Access Memory), and a ROM (Read Only Memory), and is configured to perform information processing based on programs and various types of data. The control unit 11 (CPU) is an example of a processor resource.

The storage unit 12 may be constituted of, for example, a hard disk drive, a solid state drive, or a semiconductor memory. The storage unit 12 (and RAM and ROM) are examples of a memory resource. In the present embodiment, the storage unit 12 stores various kinds of information including a model generation program 81, the eye-related data 31, the behavioral data 33, and learning result data 50.

The model generation program 81 is a program that causes the model generation apparatus 1 to execute information processing (Fig. 6 to be described later) related to machine learning of the inference model 5. The model generation program 81 includes a sequence of instructions for performing the information processing. The learning result data 50 is configured to indicate information related to the generated trained inference model 5. In the present embodiment, the learning result data 50 may be generated as a result of executing the model generation program 81. Note that as long as information for executing computational processing of the trained inference model 5 can be stored, the configuration of the learning result data 50 need not be particularly limited and may be determined as appropriate according to the embodiment. In one example, the learning result data 50 may be configured to include values of computation parameters adjusted through machine learning. In some cases, the learning result data 50 may be configured to include information indicating the structure of the inference model 5 (for example, the structure of a neural network).

The external interface 13 is configured to be connected to an external apparatus in a wired or wireless manner. For example, the external interface 13 may be a USB (Universal Serial Bus) port, a communication port, a dedicated port, or the like. The type and number of the external interface 13 may be determined as appropriate according to the embodiment. When the external interface 13 includes a communication port, the model generation apparatus 1 may execute data communication with another computer (such as the inference apparatus 2) via a network. A communication standard for the communication port may be selected at will. In addition, in the present embodiment, the model generation apparatus 1 may be connected to at least one of the sensor SA and the sensor SB via the external interface 13.

The input apparatus 14 is an apparatus used for inputting data, such as a mouse, a keyboard, or a joystick. The output apparatus 15 is an apparatus used for outputting data, such as a display or a speaker. A user can operate the model generation apparatus 1 using the input apparatus 14 and the output apparatus 15. The input apparatus 14 and the output apparatus 15 may be connected via the external interface 13. The input apparatus 14 and the output apparatus 15 may be integrally constituted of a touch panel display or the like.

The drive 16 is an apparatus for reading various kinds of information such as programs stored on a storage medium 91. At least any of the model generation program 81, the eye-related data 31, the behavioral data 33, and the learning result data 50 may be stored in the storage medium 91 instead of or in addition to being stored in the storage unit 12. The storage medium 91 is configured to store various kinds of information through electrical, magnetic, optical, mechanical, or chemical action so that the information (such as stored programs) is readable by a machine such as a computer. The model generation apparatus 1 may acquire at least any of the model generation program 81, the eye-related data 31, the behavioral data 33, and the learning result data 50 from the storage medium 91. Note that the storage medium 91 may be a disk-type storage medium such as a CD or a DVD or a non-disk-type storage medium such as a semiconductor memory (for example, a flash memory). The type of the drive 16 may be selected as appropriate according to the type of the storage medium 91. The drive 16 may be connected via the external interface 13. In addition, the storage medium 91 may include a memory resource provided in another computer such as a NAS (Network Attached Storage). When the storage medium 91 is a memory resource of another computer, the drive 16 may be omitted.

Note that with respect to the specific hardware configuration of the model generation apparatus 1, components may be omitted, replaced, or added as appropriate depending on the embodiment. For example, the control unit 11 may include a plurality of hardware processors. The hardware processors may be constituted of a microprocessor, an FPGA (field-programmable gate array), a DSP (digital signal processor), a GPU (graphics processing unit), an ASIC (application-specific integrated circuit), and the like. At least any of the external interface 13, the input apparatus 14, the output apparatus 15, and the drive 16 may be omitted. When not using the behavioral data 33, the behavioral data 33 may be omitted from the storage unit 12. The model generation apparatus 1 may be constituted of a plurality of computers. In this case, the hardware configuration of each computer may or may not be the same. In addition, the model generation apparatus 1 may be a general-purpose server, a general-purpose PC (personal computer), a tablet PC, a terminal apparatus, or the like in addition to an information processing apparatus designed specifically for the services provided.

### (Inference Apparatus)

Fig. 3 schematically shows an example of a hardware configuration of the inference apparatus 2 according to the present embodiment. In one example of the present embodiment, the inference apparatus 2 is a computer in which a control unit 21, a storage unit 22, an external interface 23, an input apparatus 24, an output apparatus 25, and a drive 26 are electrically connected.

The control unit 21 to the drive 26 of the inference apparatus 2 and a storage medium 92 may be configured in a similar manner to the control unit 11 to the drive 16 of the model generation apparatus 1 and the storage medium 91 described above. The control unit 21 (CPU) is an example of a processor resource of the inference apparatus 2, and the storage unit 22 (and RAM and ROM) are examples of a memory resource of the inference apparatus 2. In the present embodiment, the storage unit 22 stores various kinds of information including an inference program 82 and the learning result data 50.

The inference program 82 is a program that causes the inference apparatus 2 to execute information processing (Fig. 7 to be described later) related to inference of substance perceived from content. The inference program 82 includes a sequence of instructions for performing the information processing. The learning result data 50 may be managed separately from the inference program 82 or may be incorporated into the inference program 82. At least any of the inference program 82 and the learning result data 50 may be stored in the storage medium 92 instead of or in addition to being stored in the storage unit 22. The inference apparatus 2 may acquire at least any of the inference program 82 and the learning result data 50 from the storage medium 92.

When the external interface 23 includes a communication port, the inference apparatus 2 may execute data communication with another computer (such as the model generation apparatus 1) via a network. In addition, the inference apparatus 2 may be connected to at least one of the sensor SC and the sensor SD via the external interface 23. An operator can operate the inference apparatus 2 using the input apparatus 24 and the output apparatus 25.

Note that with respect to the specific hardware configuration of the inference apparatus 2, components may be omitted, replaced, or added as appropriate depending on the embodiment. For example, the control unit 21 may include a plurality of hardware processors. The hardware processor may be constituted of a microprocessor, a FPGA, a DSP, a GPU, an ASIC, or the like. At least any of the external interface 23, the input apparatus 24, the output apparatus 25, and the drive 26 may be omitted. The inference apparatus 2 may be constituted of a plurality of computers. In this case, the hardware configuration of each computer may or may not be the same. The inference apparatus 2 may be a general-purpose server, a general-purpose PC, a tablet PC, a terminal apparatus, or the like in addition to an information processing apparatus designed specifically for the services provided.

### [Software Configuration]

### (Model Generation Apparatus)

Fig. 4 schematically shows an example of a software configuration of the model generation apparatus 1 according to the present embodiment. The control unit 11 of the model generation apparatus 1 deploys the model generation program 81 stored in the storage unit 12 onto the RAM and executes the instructions contained in the model generation program 81 using the CPU. Accordingly, the model generation apparatus 1 operates as a computer including an acquisition unit 111, a training unit 112, and an output processing unit 113 as software modules.

The acquisition unit 111 is configured to acquire eye-related data 31 measured from the subject LU viewing the content LC. The training unit 112 is configured to use the acquired eye-related data 31 to perform machine learning of the inference model 5. The machine learning includes training the inference model 5 to acquire the ability to infer, from the eye-related data 31, a semantic representation 39 in an information space corresponding to substance included in the content LC. The output processing unit 113 is configured to output the result of machine learning.

In one example, the acquisition unit 111 may be configured to further acquire the behavioral data 33 indicating behavior of the subject LU viewing the content LC. In response thereto, the training unit 112 may be configured to use the acquired eye-related data 31 and the acquired behavioral data 33 to perform machine learning of the inference model 5. The machine learning may include training the inference model 5 to acquire the ability to infer, from the eye-related data 31 and the behavioral data 33, the semantic representation 39 in an information space corresponding to substance included in the content LC. In one example, the acquisition unit 111 may be configured to acquire the eye-related data 31 constituted of gaze data, blinking data, pupil diameter data, or a combination thereof.

### (Inference Apparatus)

Fig. 5 schematically shows an example of a software configuration of the inference apparatus 2 according to the present embodiment. The control unit 21 of the inference apparatus 2 deploys the inference program 82 stored in the storage unit 22 onto the RAM and executes the instructions contained in the inference program 82 using the CPU. Accordingly, the inference apparatus 2 operates as a computer including an acquisition unit 211, an inference unit 212, and an output processing unit 213 as software modules.

The acquisition unit 211 is configured to acquire eye-related data 41 measured from the subject TU viewing the object content TC. The inference unit 212 includes the trained inference model 5 due to storing the learning result data 50. The inference unit 212 is configured to infer, using the trained inference model 5, the semantic representation 49 in an information space corresponding to substance included in the object content TC from the acquired eye-related data 41. The output processing unit 213 is configured to output the result of inferring the semantic representation 49.

In one example, the acquisition unit 211 may be configured to further acquire the behavioral data 43 indicating behavior of the subject TU viewing the object content TC. Accordingly, the inference unit 212 may be configured to use the trained inference model 5 to infer the semantic representation 49 from the acquired eye-related data 41 and the acquired behavioral data 43. In one example, the acquisition unit 211 may be configured to acquire the eye-related data 41 constituted of gaze data, blinking data, pupil diameter data, or a combination thereof.

### (Other)

Note that the present embodiment describes an example in which the respective software modules of the model generation apparatus 1 and the inference apparatus 2 are all implemented by a general-purpose CPU. However, a part of or all of the software modules described above may be realized by one or a plurality of dedicated processors or chipsets. Each of the modules described above may be realized as a hardware module. Note that with respect to the software configurations of the model generation apparatus 1 and the inference apparatus 2, modules may be omitted, replaced, or added as appropriate depending on the embodiment.

### §3 Operation Example

### [Model Generation Apparatus]

Fig. 6 is a flow chart showing an example of a processing procedure of the model generation apparatus 1 according to the present embodiment. The following processing procedure is an example of the model generation method (information processing method) executed by a computer. However, the processing procedure of the model generation apparatus 1 is merely an example and each step may be modified as much as possible. In addition, with respect to the processing procedure described below, steps may be omitted, replaced, or added as appropriate depending on the embodiment.

### (Step S101)

In step S101, the control unit 11 operates as the acquisition unit 111 and acquires the eye-related data 31 for training measured from the subject LU viewing the content LC.

In one example, the control unit 11 may further acquire the behavioral data 33 indicating behavior of the subject LU viewing the content LC. The sensor SA may be used to acquire the eye-related data 31, and the sensor SB may be used to acquire the behavioral data 33. The generation of at least a part of the eye-related data 31 and the behavioral data 33 may be performed by the model generation apparatus 1 or by another computer. The control unit 11 may acquire at least a part of the eye-related data 31 and the behavioral data 33 via a network, an external storage apparatus, the storage medium 91, and the like. An order in which the eye-related data 31 and the behavioral data 33 are acquired need not be particularly limited and may be selected as appropriate according to the embodiment. The acquisition of the eye-related data 31 and the behavioral data 33 may be at least partially executed in parallel. In addition, in one example, the acquired eye-related data 31 may be constituted of gaze data, blinking data, pupil diameter data, or a combination thereof. Once the control unit 11 acquires the eye-related data 31, the control unit 11 advances processing to next step S102.

### (Step S102)

In step S102, the control unit 11 operates as the training unit 112 and uses the acquired eye-related data 31 to perform machine learning of the inference model 5. In the machine learning, the control unit 11 trains the inference model 5 to acquire, from the eye-related data 31, the ability to infer the semantic representation 39 in an information space corresponding to substance included in the content LC. Accordingly, the trained inference model 5 having acquired the ability to infer semantic representation from eye-related data within the scope of the training samples can be generated.

In one example, when the behavioral data 33 has been acquired, the control unit 11 may further use the acquired behavioral data 33 for the machine learning of the inference model 5. In other words, the control unit 11 may train the inference model 5 so that, when provided with the eye-related data 31 and the behavioral data 33 during processing of machine learning, the inference model 5 outputs an inference result that matches the correct answer for the corresponding semantic representation 39. Accordingly, the trained inference model 5 having acquired the ability to infer semantic representation from eye-related data and behavioral data can be generated.

In one example, training the inference model 5 may involve using training samples to optimize the values of computation parameters of the inference model 5. The method of machine learning may be determined as appropriate according to the embodiment including the type, the structure, and the like of the machine learning model to be used as the inference model 5. Any method may be adopted to adjust the operation parameters, such as backpropagation or solving an optimization problem. In addition, in one example, the control unit 11 may execute computational processing of machine learning in the model generation apparatus 1 or may cause another computer to execute the computational processing of machine learning by giving instructions to the other computer. Once the machine learning (training of the inference model 5) is completed, the control unit 11 advances processing to next step S103.

### (Step S103)

In step S103, the control unit 11 operates as the output processing unit 113 and outputs the result of the machine learning.

A destination of the output and substance of the output information may be selected as appropriate depending on the embodiment. In one example, as output processing, the control unit 11 may generate the learning result data 50 indicating the result of the machine learning and store the generated learning result data 50 in a predetermined storage area. The predetermined storage area may be, for example, the RAM in the control unit 11, the storage unit 12, an external storage apparatus, a storage medium, or a combination thereof. The storage medium may be, for example, a CD, a DVD, a semiconductor memory, or the like. The external storage apparatus may be, for example, a data server such as an NAS. The external storage apparatus may be, for example, an externally-attached storage apparatus. When the machine learning of the inference model 5 is to be executed by another computer, the learning result data 50 may be generated by the other computer. In addition, in another example, the control unit 11 may output information (such as loss) obtained during the operational process of the machine learning as output processing. The destination of the output may be, for example, the RAM in the control unit 11, the storage unit 12, the output apparatus 15, an external computer, an external storage apparatus, a storage medium, or a combination thereof.

Once output of the result of machine learning is completed, the control unit 11 ends the processing procedure of the model generation apparatus 1 according to the present operation example.

Note that the generated learning result data 50 may be provided to the inference apparatus 2 at any timing and by any method. For example, the control unit 11 may transmit the learning result data 50 to the inference apparatus 2 as the processing in step S103 described above or separate from the processing in step S103. The inference apparatus 2 may acquire the learning result data 50 (the trained inference model 5) by receiving the learning result data 50. Alternatively, for example, the inference apparatus 2 may acquire the learning result data 50 by accessing the model generation apparatus 1 or the data server via a network. Alternatively, for example, the inference apparatus 2 may acquire the learning result data 50 via the storage medium 92. Alternatively, for example, the learning result data 50 may be incorporated into the inference apparatus 2 in advance.

In addition, the control unit 11 may update or newly generate the learning result data 50 by repeatedly executing the sequence of processing from step S101 to step S103 on a regular or irregular basis. When repeating the processing, changes, corrections, additions, deletions, and the like of at least a part of the training samples used for the machine learning may be executed as appropriate. Furthermore, the control unit 11 may update the learning result data 50 stored in the inference apparatus 2 by providing the updated or newly generated learning result data 50 to the inference apparatus 2 using any method.

### [Inference Apparatus]

Fig. 7 is a flow chart showing an example of a processing procedure of the inference apparatus 2 according to the present embodiment. The following processing procedure is an example of the inference method (information processing method) executed by a computer. However, the processing procedure of the inference apparatus 2 is merely an example and each step may be modified as much as possible. In addition, with respect to the processing procedure described below, steps may be omitted, replaced, or added as appropriate depending on the embodiment.

### (Step S201)

In step S201, the control unit 21 operates as the acquisition unit 211 and acquires the eye-related data 41 measured from the subject TU viewing the object content TC.

In one example, the control unit 21 may further acquire the behavioral data 43 indicating behavior of the subject TU viewing the object content TC. The sensor SC may be used to acquire the eye-related data 41, and the sensor SD may be used to acquire the behavioral data 43. The generation of at least a part of the eye-related data 41 and the behavioral data 43 may be performed by the inference apparatus 2 or by another computer. The control unit 21 may acquire at least a part of the eye-related data 41 and the behavioral data 43 via a network, an external storage apparatus, the storage medium 92, and the like. An order in which the eye-related data 41 and the behavioral data 43 are acquired need not be particularly limited and may be selected as appropriate according to the embodiment. The acquisition of the eye-related data 41 and the behavioral data 43 may be at least partially executed in parallel. In addition, in one example, the acquired eye-related data 41 may be constituted of gaze data, blinking data, pupil diameter data, or a combination thereof. Once the control unit 21 acquires the eye-related data 41, the control unit 21 advances processing to next step S202.

### (Step S202)

In step S202, the control unit 21 operates as the inference unit 212 and infers, using the trained inference model 5, the semantic representation 49 in an information space corresponding to substance included in the object content TC from the acquired eye-related data 41.

In one example, when the behavioral data 43 has been acquired, the control unit 21 may use the trained inference model 5 to infer the semantic representation 49 from the acquired eye-related data 41 and the acquired behavioral data 43.

The computational processing of the trained inference model 5 may be executed as appropriate according to the embodiment including the type, the configuration, and the like of the inference model 5. For example, when the inference model 5 is constituted of a neural network, the control unit 21 may input the eye-related data 41 (and behavioral data 43) into the trained inference model 5 and execute forward computational processing of the trained inference model 5. Accordingly, the control unit 21 can obtain, from the trained inference model 5, output corresponding to the result of inferring the semantic representation 49. Once the inference of semantic representation 49 is completed, the control unit 21 advances processing to next step S203.

### (Step S203)

In step S203, the control unit 21 operates as the output processing unit 213 and outputs the result of inferring the semantic representation 49.

The substance of information output as an inference result need not be particularly limited as long as the substance is related to the inference result of the semantic representation 49 and may be determined as appropriate according to the embodiment. In one example, the control unit 21 may output the inference result of the semantic representation 49 as is. Outputting the inference result of the semantic representation 49 as is may involve outputting a vector obtained as the semantic representation 49 due to inference and outputting substance (phrases, context, and the like) corresponding to the obtained vector. When the semantic representation has been obtained by transforming content using a predetermined projection method, the control unit 21 can obtain substance corresponding to the vector by performing an inverse transformation of the predetermined projection method on the inference result of the semantic representation 49.

In another example, the control unit 21 may execute any information processing (for example, determination processing or analysis processing) on the obtained inference result. In addition, the control unit 21 may output a result of executing the information processing as information related to the inference result.

For example, the inferred semantic representation 49 corresponds to substance perceived by the subject TU. Therefore, the control unit 21 may determine a degree of matching between the inferred semantic representation 49 (inference result of perceptual substance) and the substance actually contained in the object content TC. The control unit 21 may evaluate whether the subject TU is properly perceiving the object content TC based on the determined degree of matching. Properly perceiving may involve, for example, concentrating on viewing the content and having a clear understanding of the substance of content. When the determined degree of matching is low, the control unit 21 may evaluate that the subject TU is not properly perceiving the object content TC (not concentrating on viewing the object content TC, not having a clear understanding of the substance of the object content TC, and the like). On the other hand, when the determined degree of matching is high, the control unit 21 may evaluate that the subject TU is properly perceiving the object content TC (concentrating on viewing the object content TC, having a clear understanding of the substance of the object content TC, and the like). Whether the degree of matching is high or low may be determined, for example, by comparing the degree of matching with a threshold. The control unit 21 may output a result of the evaluation as output processing of the inference result. As one example of an applicable situation, this output form may be adopted in educational settings to evaluate whether students are concentrating on educational content or have understood the educational content.

In addition, for example, the control unit 21 may categorize the subject TU according to the obtained inference result of the semantic representation 49. Categorizing involves determining a group to which the subject TU belongs. The control unit 21 may output a result of the categorization (in other words, a result of determining a group to which the subject TU belongs) as output processing of the inference result. It is presumed that the group corresponds to perceptual tendencies. In other words, it is presumed that belonging to a same group indicates having similar perceptual tendencies. In consideration thereof, as one example of an applicable situation, this output form may be adopted in healthcare settings to infer a severity of neurological disorders that affect content perception. A result of the categorization may correspond to the severity of neurological disorders. Accordingly, medical assistance can be provided. In addition, as another example of an applicable situation, substance that is likely to attract attention in the object content TC can be estimated based on the semantic representation 49 perceived by each group. In consideration thereof, the control unit 21 may output the inferred semantic representation 49 for each group. Information of the output inference result may be used as analytical material for content production including optimizing the object content TC and creating new content. An output form as the analytical material may be adopted regardless of categorization. In other words, the control unit 21 may output the inference result of the semantic representation 49 without performing categorization. The output inference result may be used as the analytical material.

In addition, for example, a computational model that projects from the information space of semantic representation to another space (such as another latent space) may be additionally prepared. The computational model may be constituted as appropriate of, for example, a trained machine learning model or the like. The control unit 21 may transform the inference result of the semantic representation 49 into another representation by using the computational model to project the inference result of the semantic representation 49 to another space. The control unit 21 may output the obtained other representation as output processing of the inference result. The other representation may be selected at will. The other representation may be, for example, an emotional expression, a future semantic representation (future prediction), or the like. When an emotional expression is adopted as the other representation, the control unit 21 can obtain a result of inferring emotions of the subject TU during viewing of the object content TC via the inference result of the semantic representation 49. Alternatively, when a future prediction is adopted as the other representation, the control unit 21 can obtain a result of a future prediction of substance perceived by the subject TU with respect to the object content TC via the inference result of the semantic representation 49. When inferring the semantic representation 49 includes predicting the future, the other representation may be semantic representation that refers to a time further in the future than semantic representation 49. In one example, a plurality of computational models may be prepared, each of which, by being subjected to machine learning of projection relationships between the information space of semantic representation and each of a plurality of other spaces, performs projection onto each of the plurality of other spaces. By selectively using the plurality of computational models, the control unit 21 can obtain representations in any other space, starting from an information space learned through direct projected from the eye-related data.

In addition, a destination of the output need not be particularly limited and may be selected as appropriate according to the embodiment. The destination of the output may be, for example, the RAM in the control unit 21, the storage unit 22, the output apparatus 25, an external computer, an external storage apparatus, a storage medium, or a combination thereof.

Once output of the inference result is completed, the control unit 21 ends the processing procedure of the inference apparatus 2 according to the present operation example. Note that in one example, the control unit 21 may execute the sequence of processing in step S201 to step S203 in real time. In other words, the control unit 21 may execute processing of inferring the semantic representation 49 in step S202 and processing of outputting the inference result in step S203 with respect to the eye-related data 41 (and behavioral data 43) obtained in real time. In another example, the control unit 21 may retrospectively infer the semantic representation 49 with respect to the eye-related data 41 (and behavioral data 43) measured in the past. In other words, in step S201, the control unit 21 may acquire the eye-related data 41 (and behavioral data 43) measured in the past, and execute the processing of steps S202 and S203 with respect to the acquired eye-related data 41 (and behavioral data 43).

### [Features]

In the present embodiment, the model generation apparatus 1 generates the trained inference model 5 having acquired the ability to infer, from eye-related data, substance perceived from the content through the processing of step S102. By using the trained inference model 5, substance perceived by an individual while viewing content can be inferred in a simple and cost-effective manner. The inference apparatus 2 can infer substance perceived by the subject TU while viewing the object content TC in a simple and cost-effective manner through the processing of steps S201 and S202.

### §4 Modifications

While an embodiment of the present disclosure has been described in detail above, the foregoing description is merely an example of the present disclosure in every respect. It is needless to say that various improvements and modifications may be made without departing from the scope of the present disclosure. The processing steps and methods described in the present disclosure may be freely combined and implemented, provided that no technical contradictions arise.

For example, in the embodiment described above, the inference apparatus 2 stores the trained inference model 5 and executes processing of inferring the semantic representation 49. However, the entity that performs the inference processing need not be limited to the inference apparatus 2. The trained inference model 5 (learning result data 50) may be stored in another computer and the inference apparatus 2 may request the other computer to execute inference processing. In this case, the inference apparatus 2 need not store the trained inference model 5 and may acquire a result of inferring the semantic representation 49 from the other computer.

### §5 Experimental Example

The following experiment was conducted to verify whether or not it is possible to infer semantic representation of substance included in content from eye activity while viewing the content. However, it is to be understood that the present invention is not limited to the experimental example described below.

### (Preparation)

First, seven 10-minute videos were prepared. General video works were used as the videos. Subjects were asked to view the prepared videos, and the pupil diameters (Y) of the subjects while viewing the videos were measured to obtain pupil diameter data. The pupil diameter data was made up of one-dimensional numerical data for each unit time point. In addition, an individual other than the subjects (an annotator) was asked to annotate each scene in the prepared videos. A computational model (word2vec) was used to transform assigned annotations into 1,000-dimensional vectors (X_{w2v}). A known model was adopted as the computational model. Accordingly, a correct answer value of semantic representation in information space (1,000-dimensional numerical data for each unit time point) was obtained. The pupil diameters (Y) and the semantic representation vectors (X_{w2v}) were divided into training samples and evaluation samples, respectively. Specifically, an evaluation experiment in which samples obtained from six of the seven videos were used as training samples and samples from the one remaining video were used as evaluation samples was repeated seven times so that each video served as an evaluation sample, and results of the seven evaluations were then averaged to obtain evaluation results. 58 subjects participated in the present experiment.

Next, to account for delays in pupillary response, four time points were set so as to be used for estimating the pupil diameter at an object time point. For the four time points, time points of -2 seconds, +2 seconds, +6 seconds, and +10 seconds were adopted, with 0 seconds being the object time point. Then, by performing a regression analysis using the training samples, a value of each parameter (W_{w2v}) of a model (encoding model) that infers the pupil diameter data (Y) at the object time point from vectors (totaling 4,000 dimensions) of semantic representations at the four time points was determined. Equation 1 below was adopted as the regression model.
[Math.1] $Y = {X}_{w 2 v} {W}_{w 2 v} + a$ (Equation 1)

In Equation 1, a denotes a constant (noise term). The value of each 4,000-dimensional parameter obtained by the regression analysis was averaged by time points to obtain the value of each 1,000-dimensional parameter (W_{w2v_avrg}).

Next, a principal component analysis was performed with respect to the obtained values of the 1,000-dimensional parameter (W_{w2v_avrg}), and a principal component vector (W) was generated by evaluating a contribution of each vector to the inference of a pupil diameter. Dimensions of the principal component vector (W) were set to 2 to 10, 12, 14, 16, 18, and 20 dimensions. Using the principal component vector (W) for each dimension, each training sample of the semantic representation vectors (X_{w2v}) was projected onto principal component space to obtain each sample of a compressed vector (Xe_{w2v}).

Next, to account for delays in time, 21 time points were set so as to be used for inferring semantic representation at the object time point. For the 21 time points, time points from 20 seconds ago to 20 seconds in the future in 2-second increments were adopted, with 0 seconds being the object time point. Then, by performing a regression analysis using the training samples, a value of each parameter (D) of a regression model (decoding model) that infers the compressed vector (Xe_{w2v}) from the pupil diameter data (Y) at the 21 time points was determined for each dimension of the principal component vector.

Fig. 8 shows a relationship between the principal component vector and the decoding model obtained in the experimental example. As shown in Fig. 8, by projecting (inverting) the compressed vector (Xe_{w2v}) in the principal component space onto a transposed matrix (W^{t}) of the principal component vector, a restored vector (Xd_{w2v}) of semantic representation in the original 1,000-dimensional space can be obtained. In consideration thereof, using the decoding model and the transposed matrix (W^{t}), an inference model that infers a vector (X_{w2v}) of semantic representation from the pupil diameter (Y) was constructed for each dimension of the principal component vector.

### (Verification)

Next, using the evaluation samples, accuracy of the obtained inference model was evaluated for each dimension of the principal component vector and for each subject. Specifically, using the decoding model in the obtained inference model, a vector (Xe_{w2v}) of a compressed semantic representation in the principal component space was calculated from the pupil diameter (Y), and an inference accuracy (correlation coefficient) of the inference model was calculated according to whether or not the vector (Xe_{w2v}) calculated from the pupil diameter (Y) matches a vector (Xe_{w2v}) of a compressed semantic representation calculated from the original vector (X_{w2v}) using the principal component vector. In the accuracy evaluation, each of the 58 subjects was set as a first subject and the remaining 57 subjects were set as the second subject. The principal component vector to be applied to the first subject was obtained by averaging the principal component vectors obtained from the 57 second subjects. The decoding model was generated using training samples obtained from the first subject, and the generated decoding model was applied to the evaluation samples. The evaluation described above was repeated for 58 subjects for each dimension of the principal component vector, and an average inference accuracy for each dimension of the principal component vector was calculated by averaging the calculated accuracy values (Fig. 9). In addition, using the obtained inference model, a vector (Xd_{w2v}) of semantic representation was calculated from the pupil diameter (Y) obtained at each time point of the evaluation sample videos. Then, by arranging the calculated vectors (Xd_{w2v}) in a space using t-SNE (t-distributed Stochastic Neighbor Embedding), substance corresponding to the vectors (Xd_{w2v}) was represented in the space (Fig. 10).

Furthermore, to verify individual differences, the encoding model (parameter (W_{w2v})) obtained from the first subject was applied to a second subject (who was not the first subject) to evaluate whether the pupil diameter of the second subject at the object time point could be correctly inferred from the vector of semantic representation. Each of 30 subjects was set as a first subject and the remaining 29 subjects were set as the second subject. The pupil diameter of the second subject was then inferred from the encoding model of the first subject, and accuracy was evaluated based on the inference result (29 times). For each of the 30 subjects, a difference between the accuracy when inferring the pupil diameter of the first subject using the encoding model of the first subject and an average accuracy of the 29 evaluations (30 evaluations in total) was calculated to calculate a change in inference accuracy across individuals (Fig. 11). Note that a commercially-available PC as used for each of the computations described above.

### (Results)

Fig. 9 shows a result of calculating an average value of inference accuracy of the inference model in the experimental example for each dimension of the principal component vector. Fig. 10 shows a result of representing, using t-SNE, perceptual substance inferred from a pupil diameter. In addition, Fig. 11 shows a result of calculating a change in inference accuracy of an encoding model among individuals in the experimental example.

As shown in Fig. 9, for example, when the number of dimensions of the principal component vectors was three, the average accuracy (correlation coefficient) for inferring a 1,000-dimensional semantic representation from a one-dimensional pupil diameter was approximately 0.18. These results indicate that the obtained inference model yields a statistically significant increase in inferential ability (with a correlation coefficient of approximately 0.18) compared to cases where no inferential ability is present. In addition, as shown in Fig. 10, substance of content could be reconstructed based on a one-dimensional pupil diameter. Pupil diameter is one example of eye activity, and other activities of the eyes such as gaze and blinking are also related to pupil diameter. Therefore, from these results, it was found that it is possible to infer substance perceived by an individual while viewing content based on eye activity.

Furthermore, as shown in Fig. 11, the inference accuracy of the coding model deteriorated in some subjects but did not deteriorate in others. These results suggest that, although there are individual differences, eye activity is similar within at least some ranges, and that even trained inference models generated from the eye-related data of another person is capable of inferring semantic representation. Furthermore, it was found that inference results of semantic representation can be categorized, suggesting that it is possible to analyze tendencies of subjects for each group. In other words, by categorizing the inference results of semantic representation, suggestions that applications toward diagnostics, content optimization, and the like described earlier are possible were obtained.

### [Reference Signs List]

- 1: Model generation apparatus
- 11: Control unit
- 12: Storage unit
- 13: External interface
- 14: Input apparatus
- 15: Output apparatus
- 16: Drive
- 81: Model generation program
- 91: Storage medium
- 111: Acquisition unit
- 112: Training unit
- 113: Output processing unit
- LU: Subject
- LC: Content
- SA, SB: Sensor
- 31: Eye-related data
- 33: Behavioral data
- 39: Semantic representation
- 2: Inference apparatus
- 21: Control unit
- 22: Storage unit
- 23: External interface
- 24: Input apparatus
- 25: Output apparatus
- 26: Drive
- 82: Inference program
- 92: Storage medium
- 211: Acquisition unit
- 212: Inference unit
- 213: Output processing unit
- TU: Subject
- TC: Object content
- SC, SD: Sensor
- 41: Eye-related data
- 43: Behavioral data
- 49: Semantic representation
- 5: Inference model

## Claims

1. A model generation method comprising executing by a computer:
a step of acquiring eye-related data measured from a subject viewing content;
a step of using the acquired eye-related data to perform machine learning of an inference model; and
a step of outputting a result of the machine learning, wherein
the machine learning includes training the inference model so as to acquire an ability to infer, from the eye-related data, a semantic representation in an information space corresponding to substance included in the viewed content.

2. The model generation method according to claim 1, wherein
the computer further executes a step of acquiring behavioral data indicating behavior of the subject viewing the content,
the acquired behavioral data is further used for the machine learning of the inference model, and
in the machine learning, the inference model is trained to acquire the ability to infer the semantic representation from the eye-related data and the behavioral data.

3. The model generation method according to claim 1, wherein
the acquired eye-related data is constituted of gaze data, blinking data, pupil diameter data, or a combination thereof.

4. An inference program causing a computer to execute:
a step of acquiring eye-related data measured from a subject viewing object content;
a step of using a trained inference model to infer a semantic representation in an information space corresponding to substance, which is included in the object content, from the acquired eye-related data; and
a step of outputting a result of inferring the semantic representation.

5. The inference program according to claim 4, further causing a computer to execute a step of acquiring behavioral data indicating behavior of the subject viewing the object content, wherein
in the step of inferring semantic representation, the computer is caused to infer the semantic representation from the acquired eye-related data and the acquired behavioral data.
